# EUROPEAN PATENT APPLICATION

(11) **EP 1 356 874 A1**
(43) Date of publication of application: **29.10.2003**
(21) Application number: 01902678.0
(22) Date of filing: 31.01.2001
(51) Int. Cl.: B09B 3/00, C05F 9/00

(54) **ORGANIC WASTE FERMENTATIVELY-PROCESSING DEVICE**

(71) Applicant: Ueda, Yasuichi, Kunigami-gun, Okinawa 905-0212 (JP); Ueda, Hiromi, Meguro-ku, Tokyo 153-0041 (JP)
(72) Inventor: UEDA, Yasuichi, Motobu-cho, Kunigami-gun, Okinawa 905-02 (JP); UEDA, Hiromi, Meguro-ku, Tokyo 153-0041 (JP)
(74) Representative: Lorenz, Werner, Dr.-Ing.
(86) International application number: JP0100629
(87) International publication number: WO02060608

(57) **Abstract**

This invention provides an organic-waste fermentation disposing apparatus that can efficiently and in short time ferments and composts organic waste like leftover, garbage and so on.

This invention is characterized in that an organic-waste fermentation disposing apparatus that contains fermentation accelerator additives seeded with fermentation microorganisms and organic waste in a disposing tank 10, that ferments and decomposes said organic waste by said fermentation microorganisms and that is covered by insulating material 11 to prevent fermentation heat generated by the fermentation of organic waste from leaking outside is provided. In addition, it is characterized in that, in order to absorb surplus heat generated inside the disposing tank and to transfer said surplus heat to the outside of the disposing tank 10, a heat exchange system 11f, 11g, 18 is provided, or in order to resist the pressure exerted by vapor due to the fermentation of said organic waste and to maintain high temperature and high pressure inside said disposing tank, an airtight sealing system 11a, 19, 20 is provided.

## Description

### Field of the Invention

The present invention relates to an organic-waste fermentation disposing apparatus that can efficiently and in short time ferment and compost organic waste like leftover, garbage and so on.

### Background of the Invention

There have been suggestions to invent organic-waste fermentation disposing apparatuses that decompose and compost such organic waste as leftover and garbage from such facilities as restaurants, hotels, and cooking facilities at hospitals and schools by fermentation that uses certain microorganisms. In recent years, there have been suggestions to invent an organic-waste fermentation disposing apparatus that uses thermophilic bacteria.

Thermophilic bacteria increase at a higher temperature range than normal-temperature microorganisms, and by using thermophilic bacteria one can not only decompose organic matters but also inactivate pathogenic bacteria and eggs of harmful parasites in the organic waste by exposing them to high temperature.

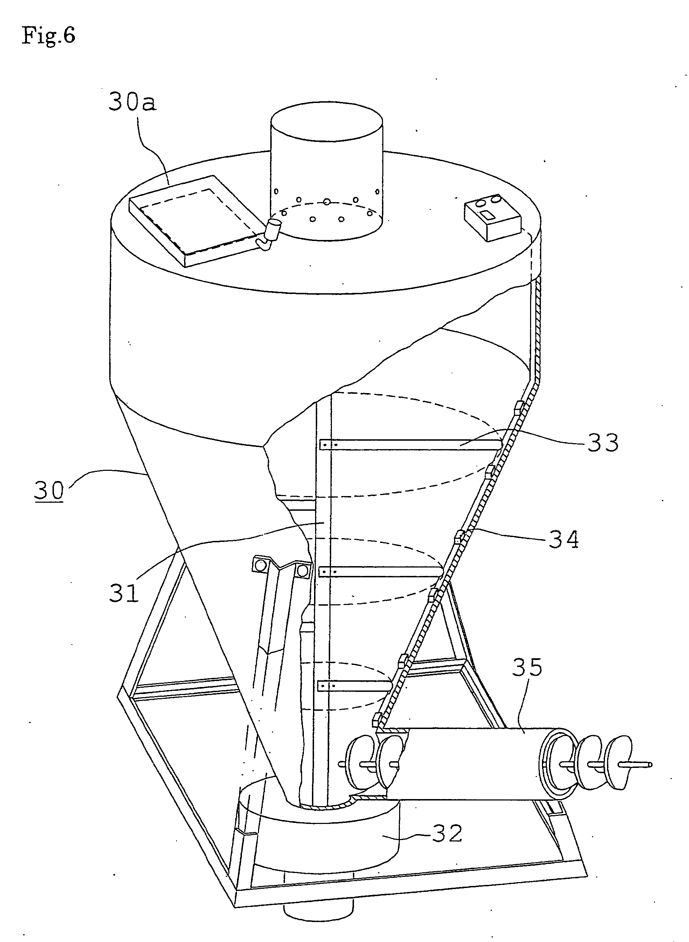

Figure 6 shows a conventional organic-waste fermentation disposing apparatus that uses thermophilic bacteria. The present inventor invented this apparatus and filed a patent application on this invention. The details are on Japan Patent Application No. JP11-199356. This organic-waste fermentation disposing apparatus ferments waste in an approximately uniform process regardless of the vertical location of the waste inside the tank, and for this reason, the tank has a substantially inverted-conical shape.

As shown in this figure, a conventional organic-waste fermentation disposing apparatus schematically is comprised of a substantially inverted-conically-shaped disposing tank 30 that can contain fermentation accelerator additives that is seeded with fermenting microorganisms and organic waste like leftover, garbage and so on, a rotating shaft 31 that vertically penetrates and is supported by the disposing tank 30, a motor 32 that is connected to the bottom end of the rotating shaft 31, a plurality of cutter blades 33 that are fixed on the rotating shaft 31 with regular vertical intervals in between and a number of thermo sensors 34 that measure the temperature at different sections inside the disposing tank 30.

This organic-waste fermentation disposing apparatus operates in the following manner.

First, a designated amount of fermentation accelerator additives that have been seeded with thermophilic bacteria in advance is placed at the bottom part of the disposing tank 30. These fermentation accelerator additives are comprised of cultures of several kinds of microorganisms (thermophilic bacteria) living in the soil and of sawdust. Then, organic waste like leftover, garbage and so on is thrown into this fermentation accelerator additives in the disposing tank 30 through a waste intake opening 30a.

When the motor 32 is operated to rotate the rotating shaft 31 at 110 times per minute, the cutter blades 33 fixed on the rotating shaft 31 crush the organic waste, and the fermentation accelerator additives and the organic waste are stirred and mixed.

Meanwhile, bacteria, fungi and actinomycetes which increase at comparatively lower temperature among a number of microorganisms living in the organic waste start fermenting, and they abruptly increase. Along with this process, the organic waste is decomposed into carbon dioxide and water, and simultaneously, the inside temperature of the disposing tank goes up to 30~65°C by the heat that is produced by this fermenting decomposition (This is called fermentation heat in this specification). When this process takes place, yeast, molds, nitrate-forming bacteria and so on, being easily affected by the heat, die out.

When the temperature goes up, thermophilic bacteria in said fermentation accelerator additives start to increase and the temperature in the disposing tank further goes up. When this happens, most pathogenic bacteria, eggs of toxic insects, parasites, virus, seeds of weeds and so on become inactivated and the organic waste become harmless to men and livestock.

In this way, the thermophilic bacteria maximizes the decomposition of the organic waste into carbon dioxide and water, and, through a screw conveyor 35 which is provided on the bottom of the disposing tank 30, homogenous, good quality compost can be obtained.

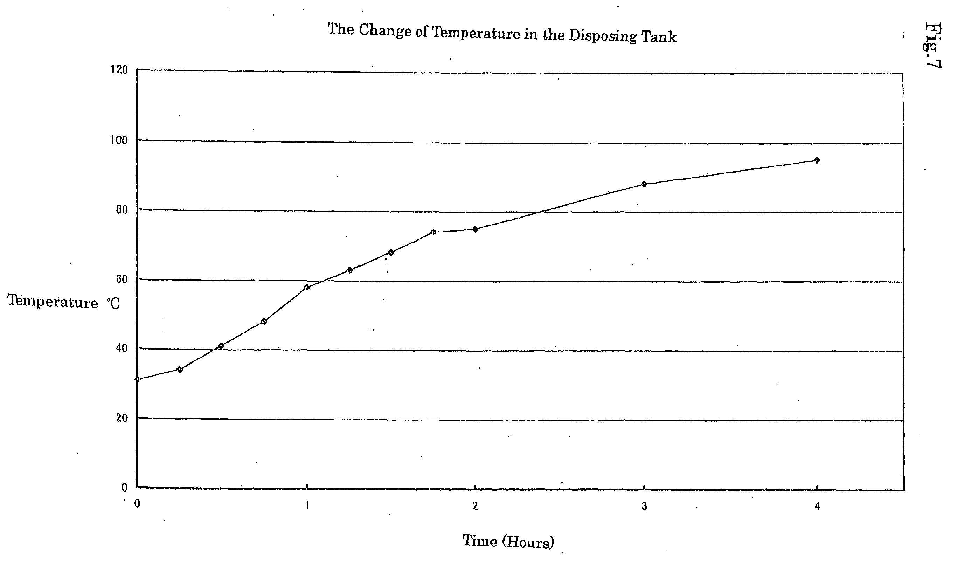

Figure 7 shows the relationship between time and the inside temperature of the disposing tank when a conventional organic-waste fermentation disposing apparatus is used for fermentation. As shown in this figure, at a conventional organic-waste fermentation disposing apparatus, fermenting process proceeds at 65~ 95°C, and in about two hours, the early stage of fermentation in the composting process completes.

However, because the conventional organic-waste fermentation disposing apparatus structurally allows the fermentation heat, which is generated by the fermentation of organic waste, to leak outside, it has a low energy efficiency problem.

Furthermore, in recent years, there have been attempts to shorten the processing time by raising the temperature higher than 100°C not by fermentation heat but by a heating device and to further increase sterilizing and deodorizing effects. However, in most cases, because the amount of contained water decreases due to the heating, the fermentation does not proceed properly and the heating only ends up in drying the organic waste. Therefore, under the present circumstances, energy efficiency is low and fermentation and decomposition by germs are not sufficiently realized.

The purpose of the present invention is to resolve these persisting problems, and aims to invent an organic-waste fermentation disposing apparatus that can efficiently and in short time ferment and compost organic waste like leftover, garbage and so on.

### Description of the Invention

To resolve the problems of the conventional organic-waste fermentation disposing apparatus, the present invention in Claim 1 is characterized in that: an organic-waste fermentation disposing apparatus, which contains fermentation accelerator additives seeded with fermenting microorganisms and organic waste and which ferments and decomposes organic waste by said fermenting microorganisms, is covered with insulating material for preventing the fermentation heat generated by the fermentation of organic waste from leaking outside, and in this way, the fermentation heat can be well utilized.

Furthermore, the present invention in Claim 2 is characterized in that: a heat exchange system that can absorb surplus heat generated in the disposing tank characterized in said Claim 1 and transfer said surplus heat to the outside of the disposing tank is provided, and this makes the outside use of the surplus heat possible.

Moreover, the present invention in Claim 3 is characterized in that: the heat exchange system in said Claim 2 is comprised of a hollow space between the disposing tank and the insulating material, a pouring duct to pour gas or liquid into said hollow space and a releasing duct to release the gas or liquid that has absorbed surplus heat.

Furthermore, the present invention in Claim4 is characterized in that: an organic-waste fermentation disposing apparatus as described in Claims 1 through 3 has an airtight sealing system that resists the pressure of the vapor that is generated by the fermentation of said organic waste and maintains high temperature and high pressure inside the disposing tank.

Moreover, the present invention in Claim 5 is characterized in that: an organic-waste fermentation disposing apparatus as described in Claim 4 has also a pressure adjusting system that can adjust the pressure inside the disposing tank.

### Brief Description of the Drawings

Figure 1 is a general perspective view showing the first preferred embodiment of an organic-waste fermentation disposing apparatus in the present invention.
Figure 2 is a chart showing the relationship between time and temperature inside the disposing tank when the fermentation is done by an organic-waste fermentation disposing apparatus related to Figure 1.
Figure 3 is a general perspective view showing the second preferred embodiment of an organic-waste fermentation disposing apparatus in the present invention.
Figure 4 is a general perspective view showing the third preferred embodiment of an organic-waste fermentation disposing apparatus in the present invention.
Figure 5 is a chart showing the relationship between time and temperature inside the disposing tank when the fermentation is done by an organic-waste fermentation disposing apparatus related to Figure 4.
Figure 6 is a general perspective view showing a conventional organic-waste fermentation disposing apparatus.
Figure 7 is a chart showing the relationship between time and temperature inside the disposing tank when the fermentation is done by an organic-waste fermentation disposing apparatus related to the Figure 6.

### Detailed Description of The Preferred Embodiments

Hereinafter, the first preferred embodiment of an organic-waste fermentation disposing apparatus in the present invention will be described in detail by referring to the drawings.

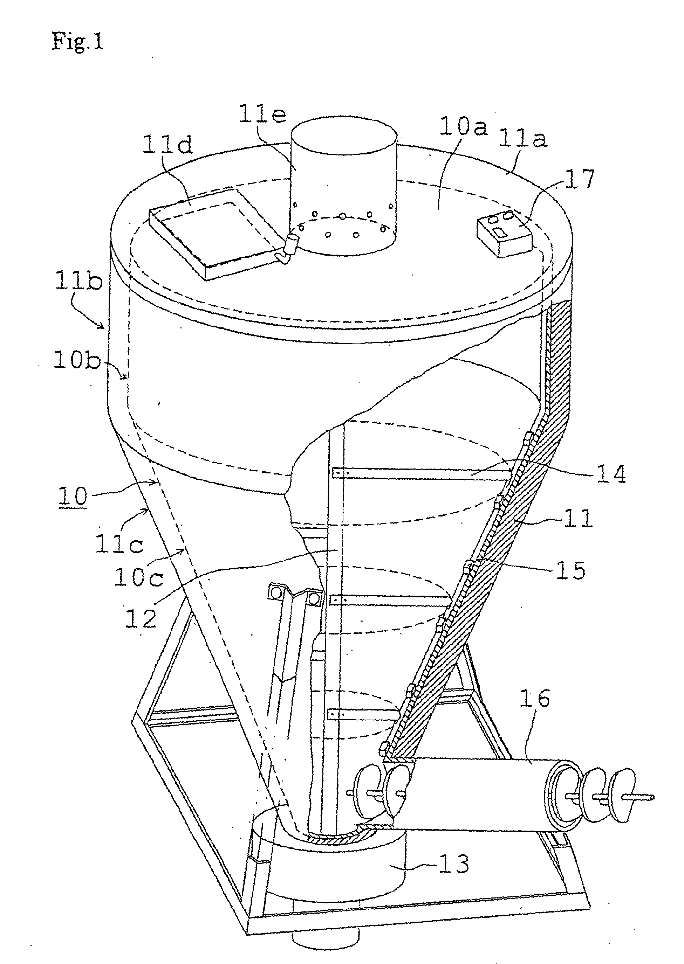

Figure 1 is a general perspective view showing an organic-waste fermentation disposing apparatus in the present preferred embodiment.

The organic-waste fermentation disposing apparatus in the present invention is schematically comprised of substantially an inverted-conically-shaped disposing tank 10, insulating material 11 covering the disposing tank 10, a rotating shaft 12 that penetrates and is supported by the disposing tank 10, a motor 13 that is connected to the bottom end of the rotating shaft 12, a plurality of cutter blades 14 that are fixed vertically on the rotating shaft 12 with regular intervals in between, a number of thermo sensors 15 that measure the temperature at different sections inside the disposing tank 10 and a screw conveyer 16 that carries processed compost outside.

The disposing tank 10 is a metallic, substantially inverted-conically-shaped container and contains fermentation accelerator additives that has been seeded with fermenting microorganisms in advance and organic waste like leftover, garbage and so on. The disposing tank 10 in the present preferred embodiment is comprised of a top face 10a, the cylinder section 10b (hereinafter referred to as cylinder), and an inverted-conical section 10c (hereinafter referred to as inverse-cone).

The outside surface of the top face 10a, the cylinder 10b and the inverse-cone 10c of said disposing tank 10 are correspondingly covered with a disc-shaped insulating material 11a (hereinafter referred to as insulating disc), a cylinder-shaped insulating material 11b (hereinafter referred to as insulating cylinder), and a inverted conically-shaped insulating material 11c (hereinafter referred to as insulating inverse-cone). In this way, because the entirety of the disposing tank 10 is covered with the insulating material 11 and the fermenting heat generated in the disposing tank 10 does not leak outside, the inside temperature of the disposing tank remains high.

On said insulating disc 11a, a control console 17 that has been programmed in advance is provided, receives signals from thermo sensors 15 inside the disposing tank and exerts control, and through this control consol, it is possible to observe the heat retention in the disposing tank 10.

Furthermore, on the insulating disc 11a, a waste intake opening 11d to take in organic waste and an exhaust duct lie to exhaust carbon dioxide gas and vapor that have been generated in the disposing tank 10 are provided.

On the rotating shaft 12, a number of cutter blades 14 like cantilevers are fixed with regular vertical intervals in between by fixing means of bolts and nuts, and these cutter blades 14 are made to rotate by the drive of the all-purpose motor 13 that is fixed to the bottom end of the rotating shaft 12. By this rotation of the cutter blades 14, the organic waste is crushed, and stirred and mixed with the fermentation accelerator additives in the disposing tank 10.

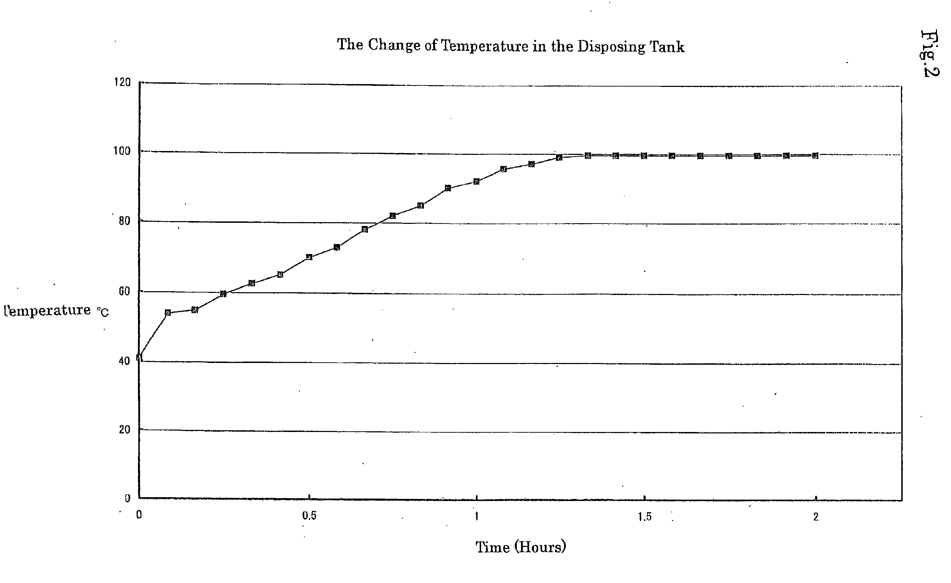

After the fermentation accelerator additives with sawdust (actual sawdust), seeded with the high-temperature thermophilic bacteria as fermenting microorganisms, had been placed in the disposing tank 10, organic waste was thrown in up to the height of about 65cm. Then, by rotating the rotating shaft 12 one hundred ten times per minute for two hours, the organic waste was crushed by the cutter blades 14 that were fixed to the rotating shaft 12 and the fermentation accelerator additives and the organic waste were stirred and mixed. After these fermentation accelerator additives and organic waste had been well crushed and mixed, the inside temperature of the disposing tank was measured by the thermo sensors 15 and the result of the measurement is shown in Figure 2.

This chart shows that the temperature in the disposing tank rapidly rose right after the measurement was taken and, in about 30 minutes, the inside temperature of the disposing tank (hereinafter referred to as inside temperature) reached 65°C. In 60 minutes, it reached the natural composting temperature of organic waste, and finally the inside temperature reached about 100°C.

This was supposedly because the fermenting heat that has been generated inside the disposing tank did not leak outside due to the insulating material 11 that covers the disposing tank 10. Because the fermenting heat in the disposing tank 10 did not leak outside, the inside temperature rose more rapidly than usual (in reference to Fig.6), and the maximum temperature reached 100°C. In this way, as a result of the rapidly rising inside temperature and the rising of the maximum temperature, fermentation that has taken two hours before can now be completed in one hour, about a half of the previous duration.

Next, the second preferred embodiment of the present invention will be described in detail by referring to the drawings. For the things and processes that are not explained, they are the same as the first preferred embodiment.

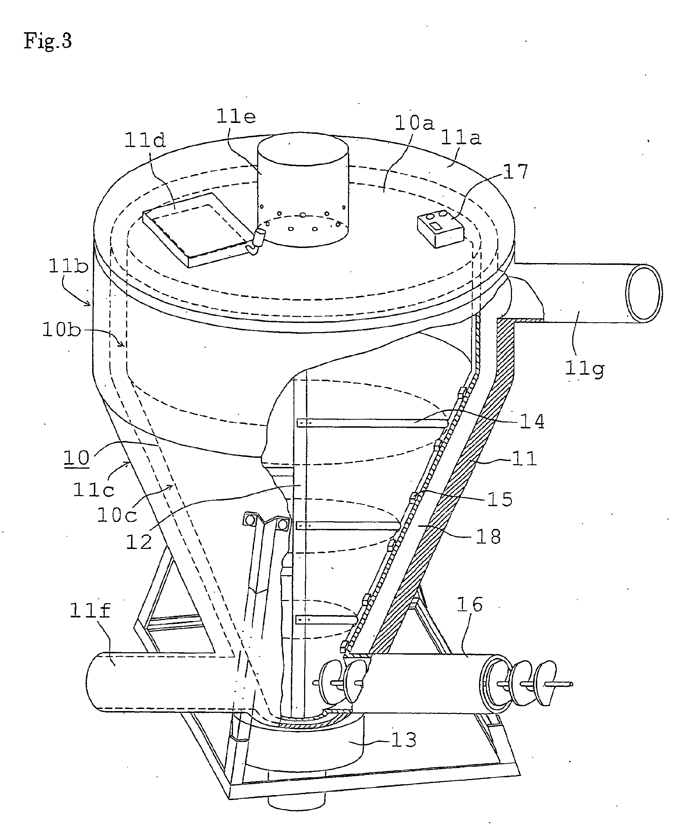

Figure 3 is a general perspective view showing an organic-waste fermentation disposing apparatus in the present preferred embodiment.

In this preferred embodiment too, the outside surface of the disposing tank 10 is covered with the insulating material 11, same as the first preferred embodiment, but it differs from the first preferred embodiment that it has a hollow space 18 between the disposing tank 10 and the insulating material 11 that covers the disposing tank 10. And at the bottom part of the inverted-conical insulator 11c of the insulator 11, a pouring duct 11f that leads to the hollow space 18 is provided, and at the upper part of the insulating cylinder 11b, a releasing duct 11g that leads to the hollow space 18 is provided.

By the way, it is known that the fermentation in the disposing tank 10 proceeds well if the temperature is higher than 65°C.

As can be seen from the chart that shows the relationship between time and the inside temperature of the first preferred embodiment (Figure 2), when the disposing tank 10 is covered with the insulating material 11, the inside temperature becomes higher than 65°C in 30 minutes. Accordingly, at this moment, in the disposing tank 10 there has generated more heat than what is needed for fermentation (in this specification, the heat more than the heat needed for fermentation is referred to as surplus heat).

In this form of the preferred embodiment, said hollow space 18, pouring duct 11f, releasing duct 11g are provided. Gas or liquid is poured into the hollow space 18 through the pouring duct 11f; and the said poured gas or liquid absorbs the surplus heat in the disposing tank 10. When the gas or liquid that absorbs the surplus heat is released outside through the releasing duct 11g, the surplus heat can be made good use of. For example, the surplus heat can be used for a heating system, warm ventilation, warm water supply and so on, and this in turn promotes recycling efficiency.

In this way, by the hollow space 18, pouring duct 11f, releasing duct 11g and the gas or liquid that is poured into by the pouring duct 11f, the surplus heat in the heat generated inside the disposing tank 10 can be absorbed and the heat exchange system such that the surplus heat in the disposing tank 10 can be transported outside is realized.

In order to release the surplus heat while maintaining necessary heat in the disposing tank 10, the amount of gas or liquid to be poured into the hollow space 18 can be adjusted as seen fit.

Next, the third preferred embodiment of the present invention will be described in detail by referring to the drawings. For the things and processes that are not explained, they are the same as the first preferred embodiment.

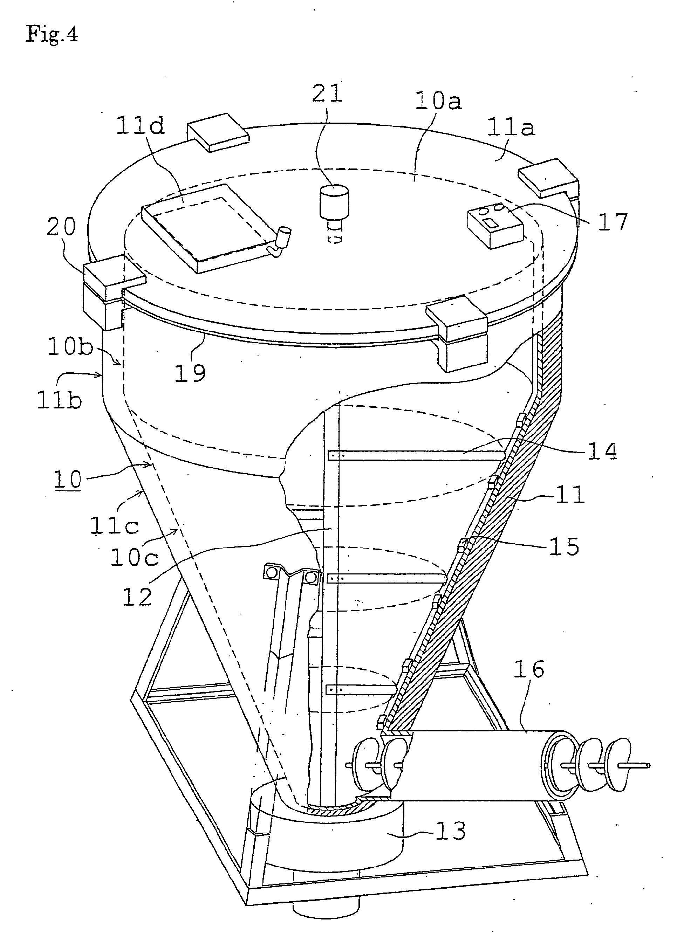

Figure 4 is a general perspective view showing an organic-waste fermentation disposing apparatus in the present preferred embodiment.

The insulating disc 11a in this preferred embodiment has a longer diameter than that of the insulating cylinder 11b that is locating below it. At the circumference of the top section of the insulating cylinder 11b, an extending edge 19 equivalent to the circumference of said insulating disc 11a is provided. Furthermore, between the insulating disc 11a and the extending edge 19, packing that is not shown in the figure is inserted.

Additionally, four vices 20 are provided on the insulating disc 11a and the extending edge 19. These vices 20 tightly press the circumference of the insulating disc 11a and said extending edge 19 against each other so that the disposing tank 10 is sealed completely airtight.

When the fermentation proceeds in the disposing tank 10 and the inside temperature becomes higher than 100°C, vapor is generated in the disposing tank 10, but because the inside of the disposing tank 10 is made airtight by said insulating disc 11a, extending edge 19a, packing and vices 20, the temperature and pressure inside the disposing tank are kept high. In other words, the insulating disc 11a, extending edge 19a, packing and vices 20 achieve an airtight sealing system that resists the pressure of the vapor generated by the fermentation of organic waste and maintain high temperature and high pressure inside the disposing tank.

The releasing duct is not provided on the insulating disc 11a in the present preferred embodiment and, instead, a pressure-adjusting valve 21 to adjust the pressure is provided. This pressure-adjusting valve 21 is of the same structure as a pressure-adjusting valve that is used in an ordinary pressure cooker, and it can adjust the inside pressure of the disposing tank 10.

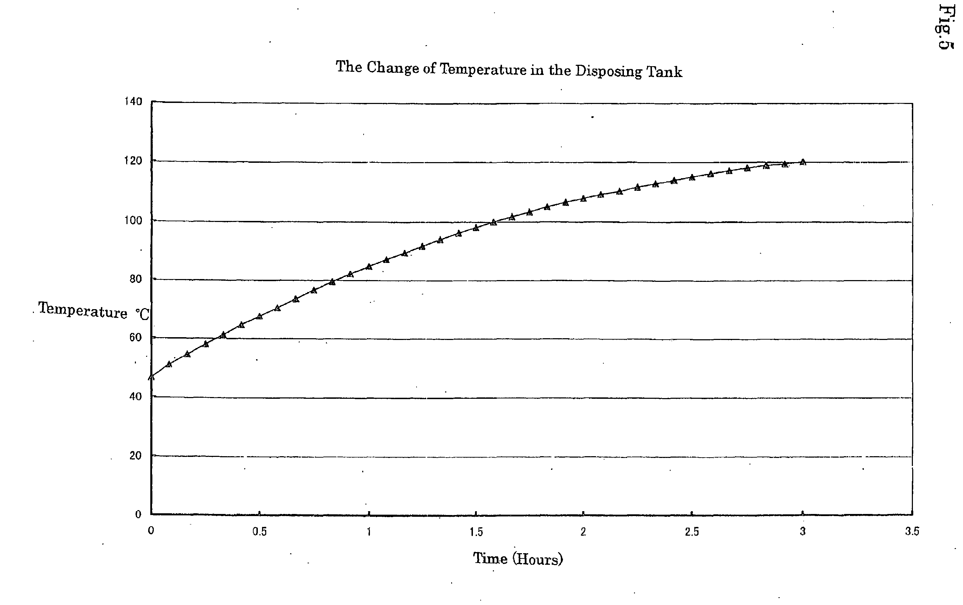

At the present disposing tank 10, the inside temperature that was measured under the same condition as the measurement for the first preferred embodiment is shown in Figure 5.

This chart shows that the temperature in the disposing tank rapidly rose right after the measurement was taken and, in about 30 minutes, the inside temperature reached 65°C. In about 90 minutes, the inside temperature rose beyond 100°C. This was supposedly because, given that said airtight sealing system is provided, high temperature and high pressure are maintained inside the disposing tank. Due to the rise of the pressure inside the disposing tank 10, the inside temperature more rapidly rose than usual (with reference to Figure 6) and the maximum temperature reached 120°C. According to the Society for Microbiology, "The temperature in which hyper thermophilic bacteria can grow ascertained for the present is about 110°C," the obtained temperature is well over the mentioned 110°C. Besides, in addition to sufficiently sterilizing and deodorizing, this apparatus provides functions of a new fermenting and decomposing apparatus or a hyper thermophilic bacteria growing apparatus.

Here, this invention is not confined to said preferred embodiments but can be embodied in a number of different forms within the scope of its technical ideas, and these different forms will be explained in the following.

The form of the disposing tank is not confined to what is shown in the figures, but can be implemented in many kinds of disposing tanks. For example, it can be applied not only to an organic-waste fermentation disposing apparatus that has an inverted-conically-shaped disposing tank, but also to those that have a cylindrical disposing tank or a horizontally-lying disposing tank.

In addition, it is not essential that insulating material be provided on the outside surface of the disposing tank, and as long as the heat does not leak outside, insulating material can be provided inside.

Furthermore, the heat exchange system is not confined to that of the second preferred embodiment; for example, a heat exchange chamber can be provided inside the disposing tank. Furthermore, it is obvious that the airtight sealing system is not limited to the use of vices and packing but could be achieved by other means.

Moreover, said third embodiment can be combined with the second embodiment. In other words, embodiments may have both the airtight sealing system and the heat exchange system.

### Industrial applicability

As stated above, under this invention, at an organic-waste fermentation disposing apparatus, because the fermentation heat generated from the fermentation of organic waste does not leak outside due to the coverage by the insulating material, high temperature is maintained inside the disposing tank, so that the fermentation is enhanced and the duration required for composting can be shortened. Furthermore, because the fermentation heat generated from fermentation is utilized, energy efficiency is high and it is unnecessary to provide an additional heating device.

Furthermore, by providing hollow space between the disposing tank and the insulating material, the pouring duct to pour gas or liquid into the hollow space and the releasing duct to release to the outside the gas or liquid that has absorbed the surplus heat, a heat exchange method in which the surplus heat can be transferred outside the disposing tank is established so that the surplus heat can be used outside and it in turn promotes an efficient recycling process.

In addition, with the airtight sealing system in which, against the pressure of the vapor generated by the fermentation of organic waste, high temperature and high pressure are maintained inside the disposing tank, not only the fermentation is greatly enhanced and the duration for composting is shortened but also the sterilizing and deodorizing effects become greater.

Moreover, with the pressure adjusting method that can adjust the inside pressure of the disposing tank, it becomes easer to adjust pressure in the fermentation process.

Furthermore, with the pressure adjusting method that can adjust the inside pressure of the disposing tank, it becomes possible to adjust the temperature to a wide range so that not only decomposition of organic waste but also incubation of microorganisms that grow at a certain temperature range during the fermentation process become possible.

## Claims

1. An organic-waste fermentation disposing apparatus that contains fermentation accelerator additives seeded with fermentation microorganisms and organic waste in the disposing tank and ferments and decomposes said organic waste by said fermentation microorganisms is **characterized in that**:
In order to prevent fermentation heat that has been generated by the fermentation of said organic waste from leaking outside, said organic-waste fermentation disposing apparatus is covered by insulating material.

2. The organic-waste fermentation disposing apparatus mentioned in Claim 1 is **characterized in that**:
Heat exchange system is provided to absorb surplus heat that has been generated in said disposing tank and transfer said surplus heat to the outside of the disposing tank.

3. The organic-waste fermentation disposing apparatus mentioned in Claim 2 is **characterized in that**:
To realize said heat exchange system, hollow space between said disposing tank and the insulating material, a pouring duct to pour gas or liquid into said hollow space and a releasing duct to release to the outside the gas or liquid that has absorbed surplus heat are provided.

4. The organic-waste fermentation disposing apparatus mentioned in Claims 1 through 3 is **characterized in that**:
An airtight sealing system to resist the pressure exerted by vapor due to the fermentation of said organic waste and maintain high temperature and high pressure inside said disposing tank.

5. The organic-waste fermentation disposing apparatus mentioned in Claim 4 is **characterized in that**:
A pressure adjusting system is provided to adjust the pressure inside said disposing tank.

## Amended claims

### Amended claims under Art. 19.1 PCT

**1.** (Amended) An organic-waste fermentation disposing apparatus that contains fermentation accelerator additives seeded with fermentation microorganism and organic waste in the disposing tank and ferments and decomposes said organic waste by said fermentation microorganism is **characterized in that**:
In order to prevent fermentation heat that has been generated by the fermentation of said organic waste from leaking outside, said organic-waste fermentation disposing apparatus is covered by insulating material, and in order to resist the pressure exerted by vapor due to the fermentation of said organic waste and maintain high temperature and high pressure inside said disposing tank, said organic-waste fermentation disposing apparatus is provided with an airtight sealing system.

**2.** The organic-waste fermentation disposing apparatus mentioned in Claim 1 is **characterized in that**:
Heat exchange system is provided to absorb surplus heat that has been generated in said disposing tank and transfer said surplus heat to the outside of the disposing tank.

**3.** The organic-waste fermentation disposing apparatus mentioned in Claim 2 is **characterized in that**:
To realize said heat exchange system, hollow space between said disposing tank and the insulating material, a pouring duct to pour gas or liquid into said hollow space and a releasing duct to release to the outside the gas or liquid that has absorbed surplus heat are provided.

**4.** (Omitted)

**5.** (Amended) The organic-waste fermentation disposing apparatus mentioned in Claim 1. Claim 2 or Claim 3 is **characterized in that**:
A pressure adjusting system is provided to adjust the pressure inside said disposing tank.

Statement under Art. 19.1 PCT

**1.** In Lines 2-4 Page 6, "... is covered with insulating material for preventing the fermentation heat generated by the fermentation of organic waste from leaking outside" is replaced with "... is covered with insulating material for preventing the fermentation heat generated by the fermentation of said organic waste from leaking outside and provided with an airtight sealing system that resists the pressure of the vapor that is generated by the fermentation of said organic waste and maintains high temperature and high pressure inside said disposing tank."
**2.** Omit Lines 14-18 Page 6.
**3.** In Claim 1 of the Claims, "covered by insulating material" is replaced with "covered by insulating material, and in order to resist the pressure exerted by vapor due to the fermentation of said organic waste and maintain high temperature and high pressure inside said disposing tank, said organic-waste fermentation disposing apparatus is provided with an airtight sealing system."
**4.** Omit Claim 4 of the Claims
**5.** In Claim 5 of the Claims, "mentioned in Claim 4" is replaced with "mentioned in Claim 1, Claim 2 or Claim 3."
